# EUROPEAN PATENT APPLICATION

(11) **EP 2 213 303 A1**
(43) Date of publication of application: **04.08.2010**
(21) Application number: 08839364.0
(22) Date of filing: 17.10.2008
(51) Int. Cl.: A61K 45/00, A61K 31/426, A61P 27/12, A61P 43/00, C07D 277/20, C07D 277/46

(54) **PHARMACEUTICAL COMPOSITION FOR TREATMENT OF CATARACT**

(30) Priority: 19.10.2007 JP 2007272993
(71) Applicant: R-Tech Ueno, Ltd., Tokyo 100-0011 (JP)
(72) Inventor: MASHIMA, Yukihiko, Tokyo 100-0011 (JP); INOUE, Ryo, Tokyo 100-0011 (JP); KISHIMOTO, Nakayuki, Tokyo 100-0011 (JP)
(74) Representative: Duckworth, Timothy John
(86) International application number: PCT/JP2008/068853
(87) International publication number: WO 2009/051223

(57) **Abstract**

The present invention aims to provide a pharmaceutical composition effective for the treatment of cataract.

A pharmaceutical composition for the treatment of cataract, containing a VAP-1 inhibitor as an active ingredient, particularly a pharmaceutical composition for the treatment of cataract, containing a compound represented by the formula (I) and the like as an active ingredient:

R¹-NH-X-Y-Z (I)

wherein each symbol is as defined in the description.

## Description

### Technical Field

The present invention relates to a pharmaceutical composition for the treatment of cataract, which comprises an effective amount of a VAP-1 inhibitor.

### Background Art

The vascular adhesion protein-1 (hereinafter to be abbreviated as VAP-1) is amine oxidase (semicarbazide sensitive amine oxidase, SSAO) abundantly existing in human plasma, which shows a remarkably increased expression in vascular endothelium and vascular smooth muscle in the inflammatory lesion. Although the physiological role of VAP-1 has not been elucidated until recently, VAP-1 gene was cloned in 1998, and VAP-1 was reported to be a membrane protein which, as an adhesion molecule, controls rolling and migration of lymphocytes and NK cells under the expression control of inflammatory cytokine. Although amine to be the substrate is unknown, it is considered to be methylamine produced in any part in the living body. It is also known that hydrogen peroxide and aldehyde produced due to the intramolecular amine oxidase activity are important factors for adhesion activity.
In addition, it is described that a thiazole derivative having a particular structure, which is a VAP-1 inhibitor, is used for the prophylaxis or treatment of VAP-1 related diseases such as macular edema, vascular hyperpermeable disease and the like (patent documents 1 - 4).
Although the VAP-1 inhibitor is known to be useful for the VAP-1 related diseases, its usefulness for the treatment of cataract is not known.
patent document 1: WO2004/067521
patent document 2: WO2004/087138
patent document 3: W02006/011631
patent document 4: W02006/028269

### Disclosure of the Invention

### Problems to be Solved by the Invention

The present invention aims to provide a pharmaceutical composition for the treatment of cataract, which comprises an effective amount of a VAP-1 inhibitor.

### Means of Solving the Problems

The present inventors have conducted intensive studies and found that a VAP-1 inhibitor is effective for the treatment of cataract, which resulted in the completion of the present invention.

Accordingly, the present invention provides the following.
[1] A pharmaceutical composition for the treatment of cataract, which comprises an effective amount of a VAP-1 inhibitor.
[2] The composition of [1], wherein the aforementioned VAP-1 inhibitor is a compound represented by the following formula (I) :

R¹-NH-X-Y-Z (I)

wherein
R¹ is acyl;
X is a divalent residue derived from optionally substituted
thiazole;
Y is a bond, lower alkylene, lower alkenylene or -CONH-;
Z is the formula

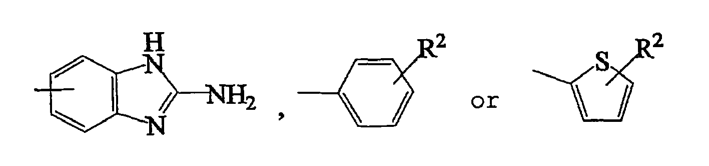

wherein R² is the formula: -A-B-D-E
wherein
A is a bond, lower alkylene, -NR^{2a}- or -SO₂- wherein R^{2a} is hydrogen, lower alkyl or acyl;
B is a bond, lower alkylene, -CO- or -O-;
D is a bond, lower alkylene, -NR^{2b}- or -CH₂NH- wherein R^{2b} is hydrogen, lower alkyl, alkoxycarbonyl or acyl; and
E is optionally substituted amino, -N=CH₂,

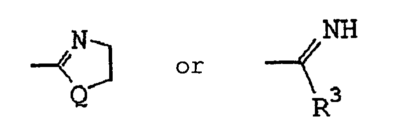

wherein
Q is -S- or -NH-;
R³ is hydrogen, lower alkyl, lower alkylthio or -NH-R⁴
wherein R⁴ is hydrogen, -NH₂ or lower alkyl,
or a derivative thereof, or a pharmaceutically acceptable salt thereof.
[3] The composition of [2], wherein, in the formula (I), Z is the formula (II):

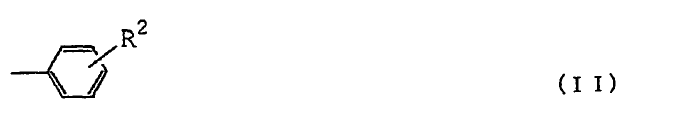

wherein
R² is the formula:

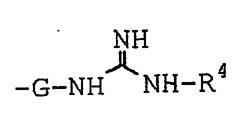

wherein G is a bond, -NHCOCH₂- or lower alkylene; R⁴ is hydrogen, -NH₂ or lower alkyl;
-NH₂; -CH₂NH₂; -CH₂ONH₂; -CH₂ON=CH₂;

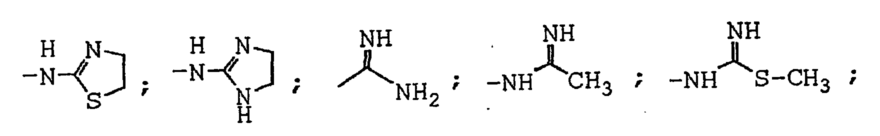

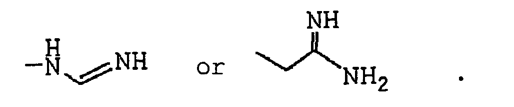

[4] The composition of [3], wherein, in the formula (II), R² is the formula:

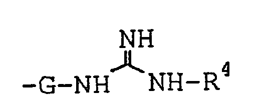

wherein G is a bond, -NHCOCH₂- or lower alkylene, and R⁴ is hydrogen, -NH₂ or lower alkyl;
-NH₂; -CH₂NH₂; -CH₂ONH₂; -CH₂ON=CH₂;

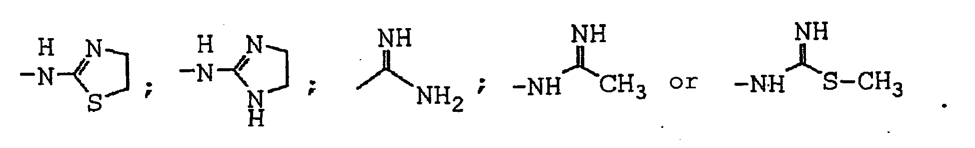

[5] The composition of [2], wherein, in the formula (I), R² is the following formula (III):
   J-L-M (III)

      wherein
      J is -NR^{2a}-, -NR^{2a}-CO-, -(CH₂)ₙ- or -(CH₂)ₙCO- wherein R^{2a} is
      hydrogen, lower alkyl, or acyl; n is an integer of 0 to 6;
      L is -NR^{2b}- wherein R^{2b} is hydrogen, lower alkyl,
      alkoxycarbonyl or acyl; and
      M is optionally substituted amino.
[6] The composition of [5], wherein, in the formula (III), J-L-M is
   -CO-NH-NH₂, -CH₂-CO-NH-NH₂, -CH₂-CO-NH-NH-CH₃, -CH₂-CO-N(CH₃)-NH₂, -CH₂-CO-NH-NH-C₂H₅, -CH₂-CO-NH-N(CH₃)₂, -(CH₂)₂-CO-NH-NH₂, -NH-CO-NH-NH₂, -NH-NH₂, -CH₂-NH-NH₂, -(CH₂)₂-NH-NH₂ or -(CH₂)₃-NH-NH₂.
[7] The composition of [2], wherein, in the formula (I), R¹ is alkylcarbonyl, and X is a divalent residue derived from thiazole optionally substituted by methylsulfonylbenzyl, and Z is the formula

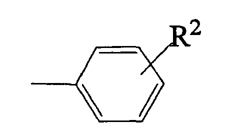

wherein R² is as defined above.
[8] The composition of [3], wherein, in the formula (I), R¹ is alkylcarbonyl, and X is a divalent residue derived from thiazole optionally substituted by methylsulfonylbenzyl.
[9] The composition of [4], wherein, in the formula (I), R¹ is alkylcarbonyl, and X is a divalent residue derived from thiazole optionally substituted by methylsulfonylbenzyl.
[10] The composition of [5], wherein, in the formula (I), R¹ is alkylcarbonyl, and X is a divalent residue derived from thiazole optionally substituted by methylsulfonylbenzyl, and Z is the formula

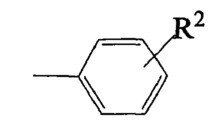

wherein R² is as defined above.
[11] The composition of [1], wherein the aforementioned VAP-1 inhibitor is N-{4-[2-(4-hydrazinocarbonylmethylphenyl)ethyl]-1,3-thiazol-2-yl}acetamide;
   or a derivative thereof; or a pharmaceutically acceptable salt thereof.
[12] The composition of [1], wherein the aforementioned VAP-1 inhibitor is
   N-(4-{2-[4-(2-{[amino(imino)methyl]amino}ethyl)phenyl]ethyl}-1,3-thiazol-2-yl)acetamide;
   or a derivative thereof;
   or a pharmaceutically acceptable salt thereof.
[13] Use of a VAP-1 inhibitor for the production of a pharmaceutical composition for the treatment of cataract.
[14] A method for treating cataract, comprising a step of administering, to a test subject in need of the treatment, a pharmaceutical composition comprising a VAP-1 inhibitor in an amount sufficient to treat the disease of the test subject.

### Effect of the Invention

The pharmaceutical composition for the treatment of cataract, which comprises an effective amount of a VAP-1 inhibitor of the present invention, can be used for the prophylaxis or treatment of the disease.

### Best Mode for Carrying out the Invention

The present invention provides a pharmaceutical composition for the treatment of cataract, which comprises an effective amount of a VAP-1 (vascular adhesion protein-1) inhibitor (hereinafter to be sometimes referred to as the composition of the present invention).
The cataract in the present invention is a disease wherein clouding develops in the crystalline lens due to various causes, resulting in vision loss. Here, cataract also includes pre-cataract stages observed as increased intensity of scattering light in the crystalline lens, colored crystalline lens, nuclear sclerosis and the like. The composition of the present invention can be used for the treatment of any cataract irrespective of the cause, particularly for the prophylaxis, namely, prevention of the onset. Such cataract includes, for example, age-related cataract, traumatic cataract, nutritional cataract, diabetic cataract, drug (steroid etc.)-induced cataract, atopic cataract, complicated cataract, radiation cataract and the like.
The term "treatment" in the present invention encompasses any management of disease including prophylaxis, treatment, relief and prevention of aggravation.

Examples of the VAP-1 inhibitor as the active ingredient of the composition of the present invention include a compound represented by the following formula (I), a derivative thereof, and a pharmaceutically acceptable salt thereof.

Formula (I):

R¹-NH-X-Y-Z (I)

wherein
R¹ is acyl;
X is a divalent residue derived from optionally substituted thiazole;
Y is a bond, lower alkylene, lower alkenylene or -CONH-; Z is the formula

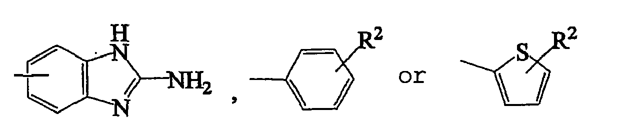

wherein R² is the formula: -A-B-D-E
wherein
A is a bond, lower alkylene, -NR^{2a}- or -SO₂- wherein R^{2a} is hydrogen, lower alkyl or acyl;
B is a bond, lower alkylene, -CO- or -O-;

D is a bond, lower alkylene, -NR^{2b}- or -CH₂NH- wherein R^{2b} is hydrogen, lower alkyl, alkoxycarbonyl or acyl; E is optionally substituted amino, -N=CH₂,

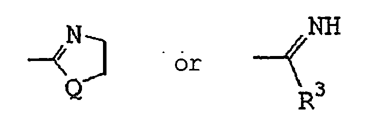

wherein Q is -S- or -NH-; R³ is hydrogen, lower alkyl, lower alkylthio or -NH-R⁴ wherein R⁴ is hydrogen, -NH₂ or lower alkyl.

The terms used for the present invention in the above- and below-mentioned descriptions of the present specification are explained in detail in the following.

The term "lower" is used to mean a group having a carbon number of 1 to 6, preferably 1 to 4, unless otherwise specified.

Examples of the "lower alkyl" include a straight chain or branched chain alkyl having a carbon number of 1 to 6 (e.g., methyl, ethyl, propyl, isopropyl, butyl, isobutyl, sec-butyl, tert-butyl, pentyl, tert-pentyl and hexyl) and the like. Among these, C₁-C₄ alkyl is more preferable.

Examples of the "lower alkylene" include a straight chain or branched chain alkylene having a carbon number of 1 to 6 (e.g., methylene, ethylene, trimethylene, tetramethylene, propylene, ethylidene and propylidene) and the like. Among these, C₁-C₄ alkylene is more preferable.

Examples of the "lower alkenylene" include a straight chain or branched chain alkenylene having a carbon number of 2 to 6 (e.g., -CH=CH-, -CH₂-CH=CH-, -CH₂-CH=CH-CH₂-, -CH₂-CH₂-CH=CH-, -CH=CH-CH=CH-, -CH=CH-CH₂-CH₂-CH₂-, -CH=CH-CH=CH-CH₂-CH₂- and -CH=CH-CH=CH-CH=CH-) and the like. Among these, C₂-C₄ alkenylene is more preferable.
The above-mentioned lower alkenylene may be an E-form or Z-form. When the compound of the present invention has a lower alkenylene moiety, the compound of the present invention encompasses any stereoisomer wherein the lower alkenylene moiety is an E-structure or Z-structure.

The "lower alkylthio" is a group wherein a sulfur atom is bonded to the alkyl moiety, which is straight chain or branched chain, the above-mentioned lower alkyl group having a carbon number of 1 to 6, and examples thereof include methylthio, ethylthio, propylthio, isopropylthio, butylthio, isobutylthio, sec-butylthio, tert-butylthio, pentylthio, tert-pentylthio, hexylthio and the like.

Examples of the "alkylcarbonyl" include alkylcarbonyl wherein the alkyl moiety has a carbon number of 1 to 6 [that is, the alkyl moiety is C₁-C₆ alkyl of the above-mentioned "lower alkyl"] (e.g., acetyl, propionyl, butyryl, isobutyryl, valeryl, isovaleryl, pivaloyl, hexanoyl and heptanoyl) and the like.

Examples of the "aryl" include C₆-C₁₀ aryl (e.g., phenyl and naphthyl) and the like, where the "aryl" may be substituted by 1 to 3 substituents and the position of substitution is not particularly limited.

Examples of the "aralkyl" include aralkyl wherein the aryl moiety has a carbon number of 6 to 10 [that is, the aryl moiety is C₆-C₁₀ aryl of the above-mentioned "aryl"], and the alkyl moiety has a carbon number of 1 to 6 [that is, the alkyl moiety is C₁-C₆ alkyl of the above-mentioned "lower alkyl"] (e.g., benzyl, phenethyl, 1-naphthylmethyl, 2-naphthylmethyl, 3-phenylpropyl, 4-phenylbutyl and 5-phenylpentyl) and the like.

The "amino" of the "optionally substituted amino" may be substituted by 1 or 2 substituents, and the substituent may be a protecting group. The "optionally substituted amino" is represented by the formula -NR^{5a}R^{5b}.

Examples of R^{5a} or R^{5b} include lower alkyl, acyl, alkoxycarbonyl, aryl, aralkyl, cyclo(lower)alkyl, cyclo(lower)alkoxycarbonyl, sulfuryl, sulfinyl, phosphoryl, heterocyclic group and the like, each of which is unsubstituted or optionally substituted, and hydrogen. The lower alkyl, acyl, alkoxycarbonyl, aryl and aralkyl are as defined above or below. Examples of cyclo(lower)alkyl include cycloalkyl having a carbon atom and having a carbon number of 3 to 6 (e.g., cyclopropyl, cyclobutyl, cyclopentyl, cyclohexyl). Examples of cyclo(lower)alkoxycarbonyl include cycloalkoxycarbonyl wherein the cycloalkyl moiety has a carbon atom and having a carbon number of 3 to 6 (e.g., cyclopropyloxycarbonyl, cyclobutyloxycarbonyl, cyclopentyloxycarbonyl, cyclohexyloxycarbonyl). In addition, they may be protected according to the method described in "Protective Groups in Organic Synthesis 3rd Edition" (published by John Wiley and Sons, 1999) and the like. R^{5a} and R^{5b} may be the same or different.

Examples of the "heterocycle" include "aromatic heterocycle" and "non-aromatic heterocycle". Examples of the "aromatic heterocycle" include a 5- to 10-membered aromatic heterocycle containing, besides carbon atoms, 1 to 3 hetero atoms selected from nitrogen, oxygen and sulfur atom and the like, for example, thiophene, furan, pyrrole, imidazole, pyrazole, thiazole, isothiazole, oxazole, isoxazole, pyridine, pyridazine, pyrimidine, pyrazine and the like. Examples of the "non-aromatic heterocycle" include a 5- to 10-membered non-aromatic heterocycle containing, besides carbon atoms, 1 to 3 hetero atoms selected from nitrogen, oxygen and sulfur atom and the like, for example, pyrrolidine, imidazoline, pyrazolidine, pyrazoline, piperidine, piperazine, morpholine, thiomorpholine, dioxolane, oxazolidine, thiazolidine, triazolidine and the like.

Examples of the "acyl" include alkylcarbonyl, arylcarbonyl and the like. Examples of the "alkylcarbonyl" include alkylcarbonyl wherein the alkyl moiety has 1 to 6 carbon atoms [that is, the alkyl moiety is C₁-C₆ alkyl of the above-mentioned "lower alkyl"] (e.g., acetyl, propionyl, butyryl, isobutyryl, valeryl, isovaleryl, pivaloyl, hexanoyl and heptanoyl) and the like.

Examples of the "arylcarbonyl" include arylcarbonyl wherein the aryl moiety has 6 to 10 carbon atoms [that is, the aryl moiety is C₆-C₁₀ aryl of the above-mentioned "aryl"] (e.g., benzoyl and naphthoyl) and the like.

Examples of the "alkoxycarbonyl" include alkyloxycarbonyl, aralkyloxycarbonyl and the like.

Examples of the "alkyloxycarbonyl" include alkyloxycarbonyl wherein the alkyl moiety has a carbon number of 1 to 10 (e.g., methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, tert-pentyloxycarbonyl, hexyloxycarbonyl etc.) and the like.

Examples of the "aralkyloxycarbonyl" include aralkyloxycarbonyl wherein the aryl moiety has a carbon number of 6 to 10 [that is, the aryl moiety is C₆-C₁₀ aryl of the above-mentioned "aryl"], and the alkyl moiety has a carbon number of 1 to 6 [that is, the alkyl moiety is C₁-C₆ alkyl of the above-mentioned "lower alkyl"] (e.g., benzyloxycarbonyl, phenethyloxycarbonyl, 1-naphthylmethyloxycarbonyl, 2-naphthylmethyloxycarbonyl, 3-phenylpropyloxycarbonyl, 4-phenylbutyloxycarbonyl and 5-phenylpentyloxycarbonyl etc.) and the like.

Examples of the "divalent residue derived from the optionally substituted thiazole" include

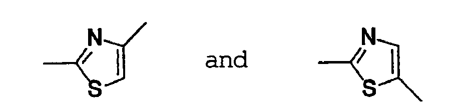

can be mentioned.

The "thiazole" may have a substituent, and the position of substitution is not particularly limited. Examples of the "substituent" of the above-mentioned "optionally substituted thiazole" include a group described in the following (1) - (12) and the like.

(1) halogen (e.g., fluorine, chlorine, bromine);

(2) alkoxycarbonyl defined above (e.g., ethoxycarbonyl);

(3) optionally substituted aryl (said aryl is as defined above, and may be substituted by -SO₂-(lower alkyl) (wherein the lower alkyl is as defined above and the like) at any substitutable position not particularly limited (e.g., phenyl and 4-(methylsulfonyl)phenyl);

(4) a group of the formula: -CONR^{a}R^{b} wherein R^{a} is hydrogen, lower alkyl, aryl or aralkyl, R^{b} is hydrogen, lower alkyl, aryl or aralkyl, where the lower alkyl, aryl and aralkyl are as defined above (e.g., N-methylaminocarbonyl, N-phenylaminocarbonyl, N,N-dimethylaminocarbonyl and N-benzylaminocarbonyl);

(5) a group of the formula: -CONH-(CH₂)ₖ-aryl wherein k is an integer of 0 to 6; aryl is as defined above, optionally has 1 to 5 substituents selected from the group consisting of -NO₂, -SO₂-(lower alkyl) wherein the lower alkyl is as defined above, -CF₃ and -O-aryl wherein aryl is as defined above, where the position of substitution is not particularly limited;

(6) a group of the formula: -CONH-(CH₂)ₘ-heterocycle wherein m is an integer of 0 to 6; and heterocycle is as defined above (e.g., pyridine);

(7) a group of the formula: -CO-heterocycle wherein heterocycle is as defined above (e.g., pyrrolidine, piperidine, piperazine, thiomorpholine), and heterocycle optionally has 1 to 5 substituents selected from the group consisting of -CO-(lower alkyl) wherein the lower alkyl is as defined above, - CO-O-(lower alkyl) wherein the lower alkyl is as defined above, -SO₂-(lower alkyl) wherein the lower alkyl is as defined above, oxo (i.e., =O) and a group of the formula: -CONR^{c}R^{d} wherein R^{c} is hydrogen, lower alkyl, aryl or aralkyl, R^{d} is hydrogen, lower alkyl, aryl or aralkyl, and lower alkyl, aryl and aralkyl are as defined above, where the position of substitution is not particularly limited;

(8) a group of the formula: -(CH₂)ₜ-aryl wherein t is an integer of 1 to 6; aryl is as defined above, and optionally has 1 to 5 substituents selected from the group consisting of -S-(lower alkyl) wherein lower alkyl is as defined above, -SO₂-(lower alkyl) wherein lower alkyl is as defined above, -SO₂-NR^{v}R^{w} wherein R^{v} is hydrogen, lower alkyl, aryl or aralkyl, R^{w} is hydrogen, lower alkyl, aryl or aralkyl, and lower alkyl, aryl and aralkyl are as defined above, -CO₂-(lower alkyl) wherein lower alkyl is as defined above, -NHCO-O-(lower alkyl) wherein lower alkyl is as defined above and a group of the formula: -CONR^{e}R^{f} wherein R^{e} is hydrogen, lower alkyl, aryl or aralkyl, R^{f} is hydrogen, lower alkyl, aryl or aralkyl, and lower alkyl, aryl and aralkyl are as defined above, where the position of substitution is not particularly limited;

(9) a group of the formula: -(CH₂)ₒ-heterocycle wherein o is an integer of 0 to 6; heterocycle is as defined above (e.g., pyrrolidine, piperidine, piperazine, morpholine, thiomorpholine), and optionally has 1 to 5 substituents selected from the group consisting of oxo (that is, =O); -CO-(lower alkyl) wherein lower alkyl is as defined above; -CO-O-(lower alkyl) wherein lower alkyl is as defined above; -SO₂-(lower alkyl) wherein lower alkyl is as defined above; -CO-(heterocycle) wherein heterocycle is as defined above (e.g., pyrrolidine, piperazine and morpholine), and optionally has 1 to 5 substituents selected from the group consisting of lower alkyl (lower alkyl is as defined above) and halogen (e.g., fluorine, chlorine, bromine), where the position of substitution is not particularly limited; and a group of the formula: -CONR^{g}R^{h} wherein R^{g} is hydrogen, lower alkyl, aryl or aralkyl, R^{h} is hydrogen, lower alkyl, aryl or aralkyl, and lower alkyl, aryl and aralkyl are as defined above, where the position of substitution is not particularly limited;

(10) a group of the formula: -(CH₂)ₚ-NRⁱR^{j} wherein p is an integer of 0 - 6; Rⁱ is hydrogen, acyl, lower alkyl, aryl or aralkyl, R^{j} is hydrogen, acyl, lower alkyl, aryl or aralkyl, and acyl, lower alkyl, aryl and aralkyl are as defined above, and lower alkyl optionally has 1 to 5 substituents selected from the group consisting of a group of the formula: -CONR^{k}R^{l} wherein R^{k} is hydrogen, lower alkyl, aryl or aralkyl, R¹ is hydrogen, lower alkyl, aryl or aralkyl, and lower alkyl, aryl and aralkyl are as defined above, where the position of substitution is not particularly limited;

(11) a group of the formula: -CON(H or lower alkyl)-(CHR^{m})_{q}-T wherein q is an integer of 0 to 6; lower alkyl is as defined above; R^{m} is hydrogen, aralkyl defined above or alkyl defined above (particularly lower alkyl), these are optionally substituted by 1 to 3 substituents selected from the group consisting of -OH and -CONH₂, where the position of substitution is not particularly limited; T is hydrogen; a group of the formula: -CONRⁿR^{o} wherein Rⁿ is hydrogen, lower alkyl, aryl or aralkyl, R^{o} is hydrogen, lower alkyl, aryl or aralkyl, and lower alkyl, aryl and aralkyl are as defined above; -NH-CO-R^{p} group wherein R^{p} is lower alkyl defined above or aralkyl defined above; -NH-SO₂-(lower alkyl) group wherein lower alkyl is as defined above; -SO₂-(lower alkyl) group wherein lower alkyl is as defined above; -heterocycle wherein heterocycle is as defined above (e.g., pyridine, pyrrolidine and morpholine), optionally has 1 to 3 substituents (e.g., oxo (that is, =O)), where the position of substitution is not particularly limited; or -CO-(heterocycle) group wherein heterocycle is as defined above (e.g., piperidine and morpholine); and

(12) a group of the formula: -(CH₂)ᵣ-CO-NR^{t}R^{u} wherein r is an integer of 1 to 6; R^{t} is hydrogen, lower alkyl, aryl or aralkyl, R^{u} is hydrogen, lower alkyl, aryl or aralkyl, and lower alkyl, aryl and aralkyl are as defined above.

The position of substitution on aryl or heterocycle may be any and is not particularly limited. Preferable "substituent" of the above-mentioned "optionally substituted thiazole" is methylsulfonylbenzyl, sulfamoylbenzyl (e.g., 4-sulfamoylbenzyl) and the like. The position of substitution of the methylsulfonyl group, sulfamoyl group and the like is not particularly limited.

Preferable example of R¹ is alkylcarbonyl (which is as defined above), and more preferable example is acetyl.
As the "divalent residue derived from thiazole" moiety of the "divalent residue derived from optionally substituted thiazole" for X,

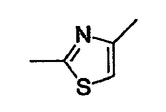

is preferable. As the "substituent" of the "divalent residue derived from optionally substituted thiazole", methylsulfonylbenzyl, sulfamoylbenzyl (e.g., 4-sulfamoylbenzyl) and the like are preferable.

Preferable examples of Y include lower alkylene (which is as defined above), and ethylene and the like are particularly preferable.

A preferable example of Z is the following formula (II):

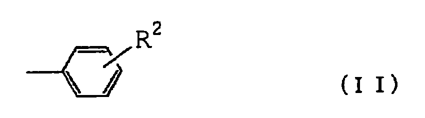

wherein R² is a group of the following formula:

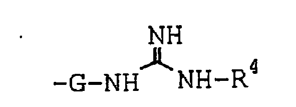

wherein G is a bond, -NHCOCH₂- or lower alkylene, and R⁴ is hydrogen, -NH₂ or lower alkyl;
-NH₂; -CH₂NH₂; -CH₂ONH₂; -CH₂ON=CH₂;

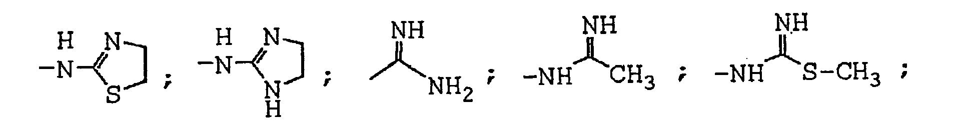

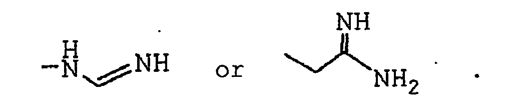

More preferably, R² is a group of the following formula:

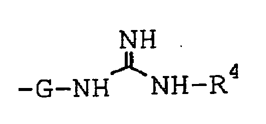

wherein G is a bond, -NHCOCH₂- or lower alkylene, and R⁴ is hydrogen, -NH₂ or lower alkyl;
-NH₂; -CH₂NH₂; -CH₂ONH₂; -CH₂ON=CH₂;

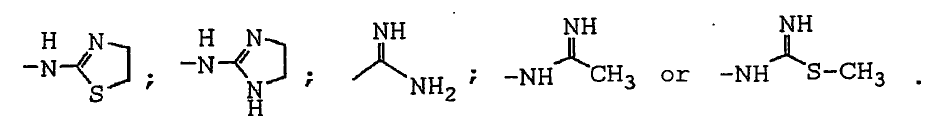

In addition, preferable examples of R² in the formula (I) include the following formula (III)

J-L-M (III)
wherein J is -NR^{2a}-, -NR^{2a}-CO-, -(CH₂)ₙ- or -(CH₂)ₙCO- (wherein
R^{2a} is hydrogen, lower alkyl, or acyl; n is an integer of 0 to 6);

L is -NR^{2b}- wherein R^{2b} is hydrogen, lower alkyl, alkoxycarbonyl
or acyl; and
M is optionally substituted amino.

Furthermore, a preferable example of the J-L-M moiety (molecular terminal) is a group wherein J is a bond, -NH-CO- or -(CH₂)ₙCO- wherein n is an integer of 0 to 2;
L is -NH- or -N(CH₃)-; and,
M is optionally substituted amino.

Specific examples of the J-L-M moiety include -CO-NH-NH₂, -CH₂-CO-NH-NH₂, -CH₂-CO-NH-NH-CH₃, -CH₂-CO-N(CH₃)-NH₂, -CH₂-CO-NH-NH-C₂H₅, -CH₂-CO-NH-N(CH₃)₂, -(CH₂)₂-CO-NH-NH₂, -NH-CO-NH-NH₂, -NH-NH₂, -CH₂-NH-NH₂, -(CH₂)₂-NH-NH_{2,} -(CH₂)₃-NH-NH₂ and the like.

In the formula (I) in the present invention, R¹ is preferably alkylcarbonyl, and X is preferably a divalent residue derived from thiazole optionally substituted by methylsulfonylbenzyl.

Moreover, the compound represented by the formula (I) in the present invention is preferably N-{4-[2-(4-hydrazinocarbonylmethylphenyl)ethyl]-1,3-thiazol-2-yl}acetamide, N-(4-{2-[4-(2-{[amino(imino)methyl]amino}ethyl)phenyl]ethyl}-1,3-thiazol-2-yl)acetamide or the like.

When the compound represented by the formula (I) in the present invention has an asymmetric carbon atom in the structure, the invention encompasses all enantiomers and diastereomers.

The "derivative" in the present invention is intended to encompass all compounds derived from the original compound.

The VAP-1 inhibitor, particularly a compound represented by the formula (I) of the present invention and a derivative thereof of the present invention can also be converted to a pharmaceutically acceptable salt. The pharmaceutically acceptable salt in the present invention is not particularly limited as long as it is a nontoxic pharmaceutically acceptable general salt, and a salt with an inorganic or organic base, acid addition salt and the like can be mentioned. Examples of the salt with an inorganic or organic base include alkali metal salt (e.g., sodium salt, potassium salt etc.), alkaline earth metal salt (e.g., calcium salt, magnesium salt etc.), ammonium salt, and amine salt (e.g., triethylamine salt, N-benzyl-N-methylamine salt etc.) and the like. Examples of the acid addition salt include salts derived from mineral acid (e.g., hydrochloric acid, hydrobromic acid, hydroiodic acid, phosphoric acid, metaphosphoric acid, nitric acid and sulfuric acid), and salts derived from organic acid (e.g., tartaric acid, acetic acid, citric acid, malic acid, lactic acid, fumaric acid, maleic acid, benzoic acid, glycol acid, gluconic acid, succinic acid and arylsulfonic acid (e.g., p-toluenesulfonic acid)) and the like.

The compound represented by the formula (I) and a derivative thereof, and a pharmaceutically acceptable salt thereof of the present invention can be used as medicaments and the like in the form of a prodrug. The term "prodrug" means any compounds that can be converted to a VAP-1 inhibitor in the body after administration. The prodrug may be any pharmaceutically acceptable prodrug of a composition of the present invention.

The compound represented by the formula (I) of the present invention and a derivative thereof, pharmaceutically acceptable salts thereof, and prodrugs thereof can be produced by a known method (W02004/067521, WO2006/011631, WO2006/028269), a combination thereof and the like.

The composition of the present invention can be administered by any route. Examples of the administration route include systemic administration (e.g., oral administration or injection administration), topical administration (e.g., instillation, eye ointment) and the like. The mode of administration of the composition of the present invention may be appropriately determined according to whether the application to cataract is for a prophylactic purpose or treatment purpose, and the like. Preferable administration route is topical administration to the eye.

The composition of the present invention is preferably administered rapidly to an administration subject such as a mammal, particularly human, after diagnosis of having a risk of cataract and before the onset thereof (prophylactic treatment). Alternatively, it is administered rapidly after the onset of cataract in the administration subject (therapeutic treatment). The treatment plan can be appropriately determined according to the kind of an active ingredient to be used, dose, administration route, cause, level of awareness of cataract where necessary, and the like.

As an administration method of the composition of the present invention, a method known per se for general medicaments can be used. The administration route may be an appropriately effective one and one or more routes can be used. Accordingly, the above-mentioned administration routes are mere exemplifications free of any limitation.

The dosage (dose) of the treatment agent of the present invention for a subject of administration such as animal including human, particularly human, is an amount sufficient to provide a desired response in the subject of administration for a reasonable period of time. The dosage is appropriately determined according to various factors including the strength of the active ingredient to be used, age, species, symptom, disease state, body weight and severity of disease of the subject of administration, the route, timing and frequency of the administration and the like. The dosage can also be appropriately controlled according to the route, timing and frequency of the administration and the like. Depending on the symptom or disease state, a long-term treatment involving plural times of administration may be necessary.

The dosage and administration schedule can be determined by a technique within the ordinary range known to those of ordinary skill in the art. In general, the prophylaxis or treatment is started from a dosage lower than the optimal dosage of the compound. Thereafter, the dosage is gradually increased until the optimal effect is obtained under the circumstances. In the composition of the present invention, the daily dosage of a VAP-1 inhibitor as an active ingredient is generally about 0.03 ng/kg body weight/day - about 300 mg/kg body weight/day, preferably about 0.003 µg/kg body weight/day - about 10 mg/kg body weight/day. Both a single administration and 2 to 4 times of administration per day can be employed before, between or after meals. In addition, the composition can be administered in a sustained manner.

The composition of the present invention preferably contains a "pharmaceutically acceptable carrier" and, as an active ingredient, a VAP-1 inhibitor in an amount sufficient to prevent or therapeutically treat cataract. The carrier may be any as long as it is generally used as a medicament, and is not particularly limited except for when it is limited by physicochemical items to be considered (e.g., solubility and lack of reactivity to the compound) and administration route.

The amount of the VAP-1 inhibitor in the composition of the present invention may vary depending on the formulation of the composition. It is generally 0.00001 - 10.0 wt%, preferably 0.001 - 5 wt%, relative to the whole composition.

The administration form (dosage form) of the composition of the present invention is not particularly limited and can be administered in various forms to achieve a desired VAP-1 inhibitory action. The composition of the present invention can be formulated into an oral or parenteral preparation by using the composition of the present invention solely or in combination with a pharmaceutically acceptable additive such as carrier, diluent and the like. The characteristics and property of the preparation are determined by the solubility and chemical property of the active ingredient, selected administration route and standard pharmaceutical practice. Examples of the preparation to be used for oral administration include solid dosage forms (e.g., capsule, tablet, powder), liquid forms (e.g., solution or suspension) and the like. Examples of the preparation to be used for parenteral administration include injection, drip and eye drop, which are in the form of a sterile solution or suspension, eye ointment and the like. The solid oral preparation may contain conventional excipients (e.g., lactose, sucrose, magnesium stearate, resin, and like materials) and the like. The liquid oral preparation can contain various aromatic, colorant, preservative, stabilizer, solubilizer, suspending agent and the like. The parenteral preparation is, for example, an aseptic aqueous or nonaqueous solution or suspension, and can contain particular various preservatives, stabilizer, buffer agent, solubilizer, suspending agent and the like. Where necessary, the solution may be made isotonic by adding an additive such as saline or glucose.

The composition of the present invention may contain other pharmaceutically active compound as long as it does not inhibit the effect of the invention.

The composition of the present invention can be administered simultaneously with other pharmaceutically active compound as long as the effect of the present invention is not impaired. The "simultaneous administration" means administration of other pharmaceutically active compound before, simultaneously (e.g., in the same preparation or in different preparation), or after administration of the composition of the present invention, by the same or different administration route. Examples of other pharmaceutically active compound include corticosteroid, prednisone, methylprednisone, dexamethasone, triamcinolone acetinide or non-corticosteroid anti-inflammatory compound (e.g., ibuprofen or flurbiprofen). Similarly, vitamin and mineral (e.g., zinc, antioxidant such as carotenoid (e.g., xanthophyll carotenoid-like zeaxanthin or lutein)), trace nutrition and the like can be recited.

The present invention provides use of a VAP-1 inhibitor for the production of a pharmaceutical composition for the treatment of cataract.

The present invention also provides a method for the treatment of cataract, which comprises a method comprising a step of administering, to a test subject in need of the treatment, a pharmaceutical composition comprising a VAP-1 inhibitor in an amount sufficient to treat the disease of the test subject or suppress the development or progression of cataract.

The present invention is explained in more detail in the following by referring to Examples (Production Example and Experimental Examples), which are not to be construed as limitative.

### Examples

The starting compounds to be used in the following Production Example can be produced by a known method (WO2004/067521, W02006/011631, WO2006/028269) and the like.

The Production Example of the VAP-1 inhibitor to be used in the present invention is shown below.

### Production Example

Synthesis of N-{4-[2-(4-hydrazinocarbonylmethylphenyl)ethyl]-1,3-thiazol-2-yl}acetamide

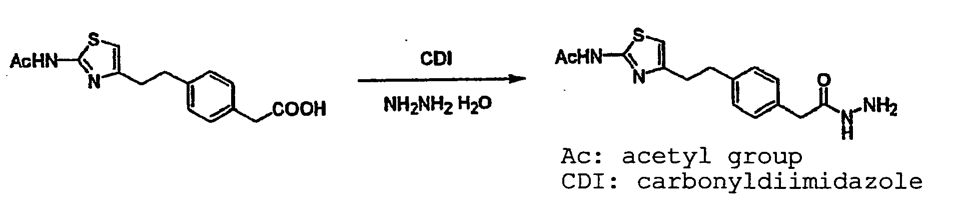

To a solution of 2-(4-{2-[2-(acetylamino)-1,3-thiazol-4-yl]ethyl}phenyl)acetic acid (913.1 mg, 3.00 mmol) in anhydrous dimethylformamide (7.5 ml) was added 1,1'-carbonyldiimidazole (729.7 mg, 4.50 mmol), and the mixture was stirred at 50°C for 1 hr. After cooling to room temperature, hydrazine monohydrate (0.73 ml, 15 mmol) was added, and the mixture was stirred at room temperature for 2 hr. Water (25 ml) was added, the mixture was stirred, and the produced solid was collected by filtration. The solid was washed three times with water, three times with ethyl acetate, and twice with tetrahydrofuran. The solid was dried under reduced pressure to give the title compound as a white solid (538.1 mg, 1.69 mmol, yield 56.3%).

melting point 200 - 202°C
¹H-NMR(200MHz,DMSO-d6): δ(ppm): 12.08(1H,brs), 9.18(1H,brs), 7.20-7.04(4H,m), 6.74(1H,s), 4.21(2H,brs), 3.40-3.25(2H,m), 3.00-2.80(4H,m), 2.11(3H,s)
¹³C-NMR(50MHz,DMSO-d6): δ(ppm): 169.8, 168.4, 157.6, 150.5, 139.6, 133.9, 129.0, 128.2, 107.5, 40.3, 34.3, 33.0, 22.7

### Experimental Example 1

Rats (Crj: Wistar, male, 5-week-old) were purchased, grouped after acclimation for 6 days, and fasted for 20 hr.
After the fasting, Streptosotocin (hereinafter STZ, 50 mg/kg (2 mL/kg)) was administered to the tail vein.
After 24 hr from the STZ administration, the blood glucose level was measured, and the rats with blood glucose level of not less than 250 mg/dL were considered as STZ induced diabetic rat (measurement tool: ACCU-CHEK Aviva, Roche Diagnostics K.K.) (group 2 - group 4).
From the next day of the blood glucose level measurement, saline, test substance 1 and test substance 2 were repetitively instilled to groups 2-4 into both eyes (10 µL for each eye) 3 times a day (9:00, 13:00, 17:00). The cataract suppression rate of the test substance at the time point when the onset of the pathology model group 2 reached 100% (Day 46) was determined. Saline was repetitively instilled in the same manner to the group free of streptosotocin administration (group 1).

In addition, the day when the nuclear part of the crystalline lens became obviously opaque was taken as the cataract onset day.

**Table 1**

| group | medicament | concentration | number of rats (eyes) |
|---|---|---|---|
| group 1 | physiological saline | - | 5(10) |
| group 2 | physiological saline | - | 5(10) |
| group 3 | test substance 1 | 0.01 wt% | 5(10) |
| group 4 | test substance 2 | 2 wt% | 5(10) |

test substance 1: N-{4-[2-(4-hydrazinocarbonylmethylphenyl)ethyl]-1,3-thiazol-2-yl}acetamide (compound described in Production Example)
test substance 2: N-(4-{2-[4-(2-{[amino(imino)methyl]amino}ethyl)phenyl]ethyl}-1,3-thiazol-2-yl)acetamide (compound described in WO2006/011631, Production Example 25)

The results are shown in Table 2.

**Table 2**

| group | number of onset eyes | onset rate (Day 46) |
|---|---|---|
| group 1 | 0/10 eye | 0% |
| group 2 | 8/8 eyes | 100% |
| group 3 | 2/8 eyes | 25% |
| group 4 | 2/10 eyes | 20% |

cataract onset rate: (number of cataract onset eyes/total number of eyes X100)

From Table 2, it is clear that the test substances 1 and 2 suppress the onset of cataract.

### Experimental Example 2

Rats (Crj: Wistar, male, 3-week-old) were purchased and, from the next day (Day 0), a mixture of a powder feed (CRF-1, Oriental Yeast Co., ltd.) and 30% of galactose (special grade, Wako Pure Chemical Industries, Ltd., Lot# 075-00035) was freely given. From Day 0, test substance 1 was repetitively administered orally. Vehicle (dissolution liquid) was administered to group 1 and test substance 1 (0.5%) was administered to group 2 at a dose of 2 mL/kg once per day, and the presence or absence of the onset of cataract was observed for 20 days.

**Table 3**

| group | medicament | concentration | number of rats (eyes) |
|---|---|---|---|
| group 1 | dissolution liquid | - | 6(12) |
| group 2 | test substance 1 | 0.5 wt% | 6(12) |

dissolution liquid: 0.5% methylcellulose solution test substance 1: N-{4-[2-(4-hydrazinocarbonylmethylphenyl)ethyl]-1,3-thiazol-2-yl}acetamide (compound described in Production Example)

The results are shown in Table 4.

**[Table 4]**

| group | number of onset eyes (Day 20) |
|---|---|
| group 1 | 8/12 eyes |
| group 2 | 3/12 eyes |

From Table 4, it is clear that test substance 1 suppresses the onset of cataract.
This application is based on a patent application No. 2007-272993 filed in Japan, the contents of which are incorporated in full herein by this reference.

## Claims

1. A pharmaceutical composition for the treatment of cataract, which comprises an effective amount of a vascular adhesion protein-1 (VAP-1) inhibitor.

2. The composition according to claim 1, wherein said VAP-1 inhibitor is a compound represented by the following formula (I):
R¹-NH-X-Y-Z (I)
wherein
R¹ is acyl;
X is a divalent residue derived from optionally substituted thiazole;
Y is a bond, lower alkylene, lower alkenylene or -CONH-;
Z is the formula 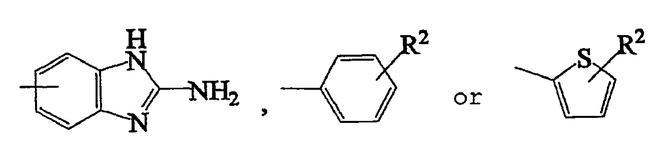 wherein R² is the formula: -A-B-D-E
wherein
A is a bond, lower alkylene, -NR^{2a}- or -SO₂- wherein R^{2a} is hydrogen, lower alkyl or acyl;
B is a bond, lower alkylene, -CO- or -O-;
D is a bond, lower alkylene, -NR^{2b}- or -CH₂NH- wherein R^{2b} is hydrogen, lower alkyl, alkoxycarbonyl or acyl; and
E is optionally substituted amino, -N=CH₂, 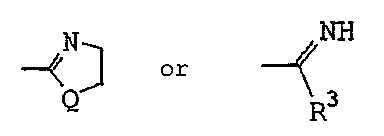 wherein
Q is -S- or -NH-;
R³ is hydrogen, lower alkyl, lower alkylthio or -NH-R⁴ wherein R⁴ is hydrogen, -NH₂ or lower alkyl,
or a derivative thereof, or a pharmaceutically acceptable salt thereof.

3. The composition according to claim 2, wherein, in the formula (I), Z is the formula (II): 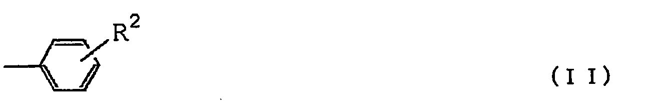 wherein
R² is the formula: 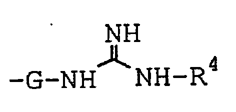 wherein G is a bond, -NHCOCH₂- or lower alkylene; R⁴ is hydrogen, -NH₂ or lower alkyl;
-NH₂; -CH₂NH₂; -CH₂ONH₂; -CH₂ON=CH₂;

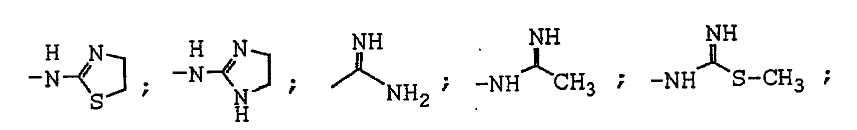

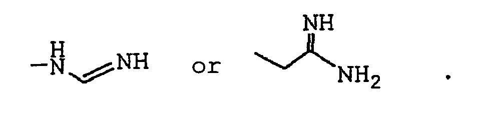

4. The composition according to claim 3, wherein, in the formula (II), R² is the formula: 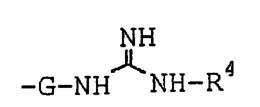 wherein G is a bond, -NHCOCH₂- or lower alkylene, and R⁴ is hydrogen, -NH₂ or lower alkyl;
-NH₂; -CH₂NH₂; -CH₂ONH₂; -CH₂ON=CH₂;

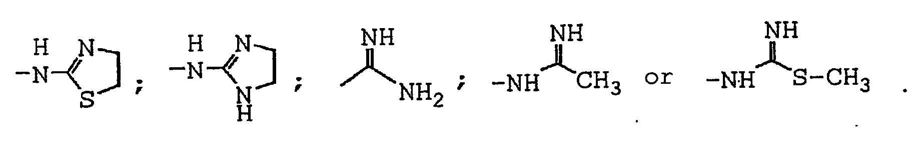

5. The composition according to claim 2, wherein, in the formula (I), R² is the following formula (III):
J-L-M (III)
wherein
J is -NR^{2a}-, -NR^{2a}-CO-, -(CH₂)ₙ- or -(CH₂)ₙCO- wherein R^{2a} is hydrogen, lower alkyl, or acyl; n is an integer of 0 to 6;
L is -NR^{2b}- wherein R^{2b} is hydrogen, lower alkyl, alkoxycarbonyl or acyl; and
M is optionally substituted amino.

6. The composition according to claim 5, wherein, in the formula (III), J-L-M is
-CO-NH-NH₂, -CH₂-CO-NH-NH₂, -CH₂-CO-NH-NH-CH₃, -CH₂-CO-N(CH₃)-NH₂, -CH₂-CO-NH-NH-C₂H₅, -CH₂-CO-NH-N(CH₃)₂, -(CH₂)₂-CO-NH-NH₂, -NH-CO-NH-NH₂, -NH-NH₂, -CH₂-NH-NH₂, -(CH₂)₂-NH-NH₂ or -(CH₂)₃-NH-NH₂

7. The composition according to claim 2, wherein, in the formula (I), R¹ is alkylcarbonyl, and X is a divalent residue derived from thiazole optionally substituted by methylsulfonylbenzyl, and Z is the formula 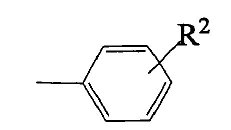 wherein R² is as defined above.

8. The composition according to claim 3, wherein, in the formula (I), R¹ is alkylcarbonyl, and X is a divalent residue derived from thiazole optionally substituted by methylsulfonylbenzyl.

9. The composition according to claim 4, wherein, in the formula (I), R¹ is alkylcarbonyl, and X is a divalent residue derived from thiazole optionally substituted by methylsulfonylbenzyl.

10. The composition according to claim 5, wherein, in the formula (I), R¹ is alkylcarbonyl, and X is a divalent residue derived from thiazole optionally substituted by methylsulfonylbenzyl, and Z is the formula 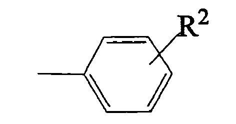 wherein R² is as defined above.

11. The composition according to claim 1, wherein said VAP-1 inhibitor is N-{4-[2-(4-hydrazinocarbonylmethylphenyl)ethyl]-1,3-thiazol-2-yl}acetamide;
or a derivative thereof;
or a pharmaceutically acceptable salt thereof.

12. The composition according to claim 1, wherein said VAP-1 inhibitor is N-(4-{2-[4-(2-{[amino(imino)methyl]amino}ethyl)phenyl]ethyl}-1,3-thiazol-2-yl)acetamide;
or a derivative thereof;
or a pharmaceutically acceptable salt thereof.

13. Use of a VAP-1 inhibitor for the production of a pharmaceutical composition for the treatment of cataract.

14. A method for treating cataract, comprising a step of administering, to a test subject in need of the treatment, a pharmaceutical composition comprising a VAP-1 inhibitor in an amount sufficient to treat the disease of the test subject.
